# EUROPEAN PATENT APPLICATION

(11) **EP 4 364 719 A1**
(43) Date of publication of application: **08.05.2024**
(21) Application number: 21947374.1
(22) Date of filing: 02.07.2021
(51) Int. Cl.: A61K 8/22, A61K 31/05, A61K 31/055, A61K 31/125, A61K 31/327, A61K 33/40, A61Q 11/00, A61P 31/00, A61P 31/02, A61P 31/04

(54) **ORAL PHARMACEUTICAL COMPOSITION FOR PREVENTING AND/OR TREATING DISEASES OF THE SOFT AND HARD TISSUES SURROUNDING THE TOOTH IN THE ORAL CAVITY**

(71) Applicant: Ingalfarma Spa, Providencia, Santiago (CL)
(72) Inventor: GALVÁN GONZÁLEZ, Tomas, Santiago (CL); ROSENBERG, David, Santiago (CL); GALVÁN INOSTROZA, Felipe, Santiago (CL)
(74) Representative: Herrero & Asociados, S.L.
(86) International application number: PCT/CL2021/050060
(87) International publication number: WO 2023/272404

(57) **Abstract**

The present invention is directed to oral pharmaceutical compositions comprising at least one statin and at least one antiseptic and/or antibacterial agent. Likewise, it is aimed at their use to prevent and/or treat diseases of the soft tissues present in the oral cavity, such as periodontal disease. A method is also described for preventing and/or treating diseases of the soft and hard tissues surrounding the tooth present in the oral cavity that comprises administering said pharmaceutical composition.

## Description

### FIELD OF THE INVENTION

The present invention relates to the field of oral medicine or dentistry, more particularly to pharmaceutical compositions useful to prevent and/or treat diseases of the soft and hard tissues surrounding the tooth present in the oral cavity, especially to prevent and/or treat periodontal disease.

### BACKGROUND

Periodontal Disease (PD) is an inflammatory-infectious and destructive condition of the tissues surrounding the tooth. Despite scientific advances and economic efforts, it continues to affect more than 90% of adults worldwide, causing tooth loss in a large part of those affected, generating enormous health expenditure and detriment to the quality of life of the population.

A series of cellular and molecular events occur in response to the accumulation of bacteria in the oral cavity. However, although bacteria are necessary to initiate PD, the immune response is responsible for the destruction of periodontal tissues.

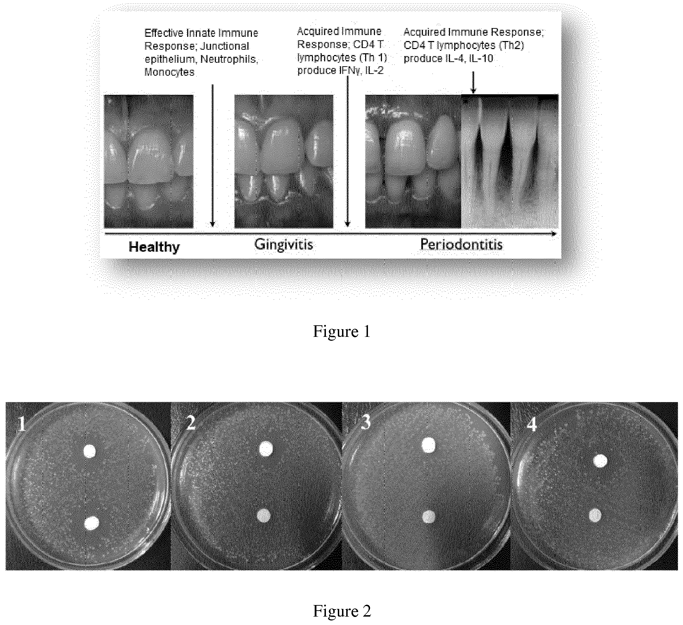

When oral hygiene measures are stopped, signs of gingival inflammation (gingivitis) progressively appear, but once oral hygiene measures are resumed, the signs of gingivitis disappear, and the gum returns to its initial state. For this reason, gingivitis is considered a reversible lesion. Without reestablishing oral hygiene, this state of reversible gingivitis can remain, in some people, chronically for an indeterminate time. However, for reasons still unknown, in some people or under certain conditions, the disease progresses to a destructive entity called periodontitis (see Figure 1).

This process takes time, thus expressing the cyclical nature of the disease, which in turn is related to other factors that can cause the inflammatory response to be modified, underlying diseases such as diabetes, habits such as smoking, and stress situations, among others, which defines it, therefore, as a multifactorial disease.

On the other hand, inflammation-infection of the periodontal tissue can affect other areas of the body, being linked to various general diseases (cardiovascular pathologies, diabetes mellitus, and premature birth, among others).

Due to the above, PD is considered a complex disease in which the development of bacteria, the host, and environmental factors participate.

According to a study that estimated the prevalence, severity, and extent of periodontitis in the US adult population, more than 47% of individuals presented periodontitis. Among them, 8.7% were diagnosed with mild periodontitis, 30% moderate, and 8.5% severe. Regarding the extent of the disease, 56% of the adult population had 5% or more periodontal sites with attachment loss ≥ 3 mm, and 18% had 5% of sites with periodontal pockets ≥ 4 mm. Periodontal disease was higher in men, Mexican Americans, adults with low economic and educational levels, and in smokers (Eke PI et al., "Prevalence of periodontitis in adults in the United States: 2009 and 2010". J Dent Res. October 2012; 91(10):914-20).

On the other hand, the prevalence of periodontal disease in Chile is higher. A study carried out in 2010 showed that 93.45% of individuals between 35 and 44 years old had an insertion loss ≥ 3 mm, and 38.65% of them had an insertion loss ≥ 6 mm in at least one place. This prevalence increased in older adults between 65 and 74 years of age, those who presented an insertion loss ≥ 3 mm in 97.58% and ≥ 6 mm in 69.35% (Gamonal J. et al., "Clinical attachment loss in Chilean adult population: First Chilean National Dental Examination Survey". J Periodontol. October 2010;81 (10): 1403-10).

From the population's health indices, it is evident that many aspects of PD are still unknown, despite Periodontics being the dental discipline that develops the most scientific knowledge.

The etiology of periodontitis is not yet fully understood since various factors have been reported to be involved in its development, such as bacterial infection, the host's immune response, and environmental factors (such as virulent pathogens, smoking, personal hygiene, and certain diseases).

Various studies have proposed two forms of periodontitis, one of them called:
a) Chronic periodontitis mainly affects the population over 35 years of age. It has been shown that this type of disease is associated with a bacterial consortium present in the subgingival area, within which are present *Fusobacterium nucleatum, Porphyromonas gingivalis, Treponema denticola,* and *Tanerella forsythia.*
b) Aggressive periodontitis, which is mainly associated with young adults. According to a study by Arowojolu et al., they found a prevalence between ages ranging from 17-34 years. However, it can develop at any age, and it has been described that it can progress 3 to 4 times faster and also affects a large percentage of adolescents. In addition to the subgingival microbial community mentioned above, this type of disease is characterized by the presence of the organism *Aggregatibacter actinomycetemcomitans,* which predominates in the affected oral sites.

Bacterial infection is considered the main etiological factor of PD. However, recent studies have focused on the immune system's role in the evolution of this pathology, indicating that bacterial antigens trigger an immunopathological reaction, where the patient's susceptibility will determine the final result of the process.

It has been postulated that while commensal bacteria are well tolerated, periodontal pathogens produce an immune response evading the host's immune control mechanisms, which ends with the activation of tissue destruction processes.

Therefore, periodontal disease is a complex multifactorial disease, in which, although bacteria are a fundamental factor in its etiology, the immune response that develops in the host is also highly relevant.

In the immunological process that occurs during the development of periodontal disease, a large number of cells and molecules participate, such as lymphocytes, neutrophils, macrophages, cytokines, interleukins, tumor necrosis factor, among others, which makes this pathology a complex process, of which there is still much to learn.

Furthermore, although some components predominate more in periodontitis and others more in gingivitis, many of them are found in both, making it difficult to understand and describe an exact mechanism of progression.

What is known is that there would be a reversible condition called gingivitis, which can remain in this state for a long time. Or, given certain immunological conditions (still unknown), gingivitis can progress to a more complex, irreversible, and destructive condition, periodontitis, which does not depend solely and exclusively on bacteria, but also immunological factors involved. Therefore, a dysfunctional immune response would be the cause of the predisposition to tissue destruction.

Understanding the etiopathogenesis of periodontal disease will allow us not only to develop mechanisms for early diagnosis of the disease, identify and explain the intrinsic and exogenous factors of periodontal risk but also to develop new preventive and therapeutic strategies, truly effective in combating one of the most prevalent diseases in the population, and provide an adequate solution to a public health problem, which still has no solution.

### DESCRIPTION OF THE PREVIOUS ART

There are different types of treatment for periodontal disease, among which the chemical control of the disease stands out.

The role and utility of topically applied chemicals have been intensively investigated in periodontics as an adjunct to mechanical procedures for plaque control and are associated with the prevention and adjunctive treatment of periodontal disease.

### Mouthwashes

Regardless of the chemical agent used, mouthwashes do not significantly penetrate the sulcus and/or periodontal pocket (0.2 mm), so their action is limited to controlling supragingival plaque and therapeutic management of gingivitis. Neither the probing depth level nor the insertion level are significantly improved.

### Chlorhexidine

It is the most researched and effective anti-plaque agent. Chlorhexidine chemically corresponds to a bisbiguanidine with cationic properties. The molecule is symmetrical, with two chlorophenyl rings and two biguanidine groups connected by a central hexamethylene chain.

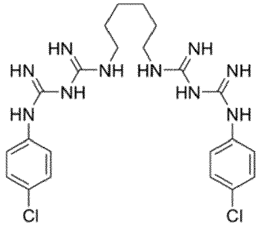

The interaction between the positively charged chlorhexidine molecule and the negative charges in the bacterial cell wall has been demonstrated. This increases cell permeability, causing a loss of osmotic balance and thus producing bacterial lysis. It also reduces the formation of the film on the tooth surface and alters bacterial adhesion.

An important property of chlorhexidine is its high substantivity, that is, the long association between a material and a substrate, even for a longer period than expected with simple mechanical deposition. This promotes the slow release of the agent to the medium.

In the case of chlorhexidine, its substantivity is 12 hours at a concentration of 0.12%. Therefore, it is considered an effective antibacterial, bactericidal in high concentrations, and bacteriostatic in low concentrations, such as those gradually diluted in saliva.

The clinical results regarding the levels of reduction of supragingival bacterial plaque and gingival inflammation are 55% and 45%, respectively. Early clinical studies used 10 mL of a 0.2% solution, equivalent to 20 mg of chlorhexidine *per* use. Currently, according to the American Dental Association (ADA) recommendation, 15 mL at 0.12%, equivalent to 18 mg of chlorhexidine, are used. The amount of agent per use is almost the same, and the clinical results are similar. Its minimum inhibitory concentration (MIC) is from 8 to 500 mg/ml, studied for 52 bacteria isolated from subgingival plaque. At a concentration of 250 mg/mL, all bacteria isolated from patients with periodontitis were inhibited. The MICs were all lower than the level achieved by topical application. Antimicrobial resistance with chlorhexidine has not been detected.

Among the adverse effects of chlorhexidine are temporary alteration of taste and an increase in staining calculus deposits (dental staining).

### First-generation chemical agents

They correspond to certain antibiotics, quaternary ammonium compounds, phenolic compounds, fluorine compounds, oxygenating agents, and povidone-iodine. Among phenolic compounds, the only product that has been studied is Listerine^{®}, which includes essential oil compositions of thymol, menthol, eucalyptol, and methyl salicylate. The alcoholic carrier of this agent reaches an extremely high concentration of 26.9% with a pH of 5.0. This explains the adverse effects associated with oral burning sensation as a result of epithelial damage, mucosal ulceration, and significant alterations in taste.

### Second-generation chemical agents

Second-generation chemical agents are characterized by high substantivity (25-30% retention after each one-minute mouth rinse). Such compounds are active in situ for hours (chlorhexidine, amine fluoride, triclosan, when combined with certain compounds). Triclosan is a non-ionic bisphenol antiseptic with low toxicity and a broad antibacterial spectrum. Because it does not bind well to oral surfaces, as it lacks a strong positive charge, formulations have been created that increase its ability to bind to plaque and tooth (combination with zinc citrate to increase its anti-plaque and anti-tartar potential, the incorporation of a methoxyethylene copolymer and maleic acid to increase its retention time). Triclosan in concentrations of 0.2-0.5% and zinc citrate in 0.5-1% promotes a significant reduction in plaque and gingivitis. The same effect has been achieved with toothpastes containing 0.3% triclosan and 0.25% copolymer of methoxyethylene and maleic acid. Mouthwash formulations at 0.3% have shown significant reductions in plaque and gingivitis. It is important to note that zinc citrate has a limited effect on bacterial growth on surfaces that have been previously cleaned but has a significant effect on areas with moderate amounts of plaque. This indicates that the main effect of zinc citrate is to reduce the rate of bacterial proliferation in already-formed plaque. On the contrary, triclosan greatly affects plaque-free surfaces (after brushing teeth) and decreases its antibacterial potential with the increase in existing plaque. This supports the hypothesis that triclosan can be adsorbed to the tooth surface, preventing bacterial adhesion or inhibiting the growth of bacteria that colonize the surface.

### Third-generation chemical agents

Those antibacterial substances with little effect but that interfere with bacterial adhesion are called third-generation agents (amino alcohols: octapinol, decapinol). Using these elements as supplements to oral hygiene measures has been shown to reduce the formation of bacterial plaque compared to a placebo mouthwash. However, from a clinical point of view, second-generation antibacterial agents remain the first choice.

### Antibiotics

Antibiotics are not indicated for the control of bacterial plaque. Their potential adverse effects exceed their possible therapeutic value and are not effective in controlling supragingival plaque or treating gingivitis.

### Irrigation devices

The use of irrigation devices can increase the ability of products to reach the subgingival area. However, several studies have concluded that, generally, irrigation at the gingival margin is not very efficient in achieving a sufficiently apical depth with respect to the subgingival plaque, even when the tip of the irrigator is placed 3 mm inside the pocket. Currently, electrical and mechanical oral irrigation devices have been designed, which have a low predictability in reaching the full depth of the gingival cleft, both in shallow and deep places.

The effectiveness of subgingival irrigation is limited by the action of crevicular fluid causing rapid clearance of the irrigant, the presence of blood components that inactivate the solution, and the presence of subgingival calculus that prevents penetration of the agent.

From a microbiological point of view, subgingival irrigation itself can temporarily alter the bacterial composition. However, within the first eight weeks, there is a complete recurrence of the original levels of subgingival bacteria. The possible reduction of motile spirochetes and Bacillus after professional irrigation may be attributed to frequent mechanical disruption rather than the effect of the agent used itself. Likewise, these microbiological changes have no effect on bleeding, attachment level, or probing depth and do not stop the progression of periodontal disease.

The clinical results (periodontitis) obtained with mechanical instrumentation (root planing) are not surpassed when subgingival irrigation is associated, regardless of the chemical agent used. The only function is mechanical drag. Except for high concentrations of chlorhexidine (2%) or tetracycline (10%) and only when applied for more than 5 minutes, adding antimicrobial agents to the irrigation solution does not add any benefit to that obtained when water alone or saline solution is used.

During the maintenance phase of a treated patient, daily irrigation can improve oral hygiene and gingival health in patients with a low level of mechanical plaque control. However, this does not mean that irrigation prevents the repopulation of the area with pathogenic bacteria or modifies the interval between maintenance sessions. For this reason, sulcal irrigation cannot replace professional mechanical instrumentation during periodontal support management.

### Toothpaste

The main functions of toothpaste (in addition to brushing) are reducing the amount of plaque, reducing the risk of cavities, removing dental stains, removing food debris, and improving breath. The benefits sought are directly influenced by the action of the main agent and its relationship with other compounds, whether from the same formulation or other sources. Toothpastes contain a series of compounds that serve the above effects:
- Abrasive agent (must clean and polish, without causing damage to the enamel, dentin, and mucous membranes; must be insoluble, inert, non-toxic, and preferably white; calcium carbonate, alumina, and silica are currently used; the size, shape, and particle hardness is important).
- Filler (provides stability and consistency; can be soluble or insoluble in water; alginates, sodium carboxymethyl cellulose, sodium magnesium, and others are used).
- Surfactant (participates in the dispersion of food and other debris; the most widely used is sodium lauryl sulfate, which also has antibacterial properties and helps solubilize key ingredients, such as flavorings and certain antibacterial agents).
- Wetting agent (helps reduce moisture loss and improves the product's texture in the mouth).
- Flavoring agents.
- Therapeutic agents (anti-cavities, anti-plaque, to reduce dentin hypersensitivity, and whitening agents).

Currently, some formulations of pastes or gels have triclosan or chlorhexidine as their main chemical agent. The effectiveness of chlorhexidine gel depends on the patient's ability to achieve effective mechanical plaque control, that is, the patient's ability to deliver the gel to appropriate sites in the mouth. Chlorhexidine gel does not easily penetrate areas far from the application site; thus, some effectiveness of the gel depends on the correct amount reaching the appropriate areas of the mouth and staying there for a long period.

There are gels with a chlorhexidine concentration of 0.1% and 1%. Considering the agent's dilution by saliva's action during brushing, the actual concentration of chlorhexidine is, therefore, lower. In conclusion, preventive measures to maintain periodontal health and therapeutic actions used to treat gingivitis and periodontitis are based on removing and controlling plaque.

The most effective plaque control is mechanical removal by practicing proper brushing and flossing technique, and professional prophylaxis.

Combining chemicals with mechanical removal for oral hygiene offers an advantage because the higher concentration of the bacterial load can be reduced mechanically, leaving a number of disorganized and thin plaque that can be removed with chemical products.

For subgingival irrigation to take an important role in the treatment of periodontal disease, it is necessary to have a significant and sustained effect on the bacterial composition, achieve a long-term positive effect on the clinical parameters of periodontitis and a beneficial effect beyond the effect achieved with root planing alone.

There are several products for the management of bacterial plaque, reduction in calculus formation, and dentin hypersensitivity. The clinical choice of these compounds must be based on scientific validity and their relationship with the patient's real needs.

### Statins

As a way to reduce the adverse effects of chemical control of bacteria, efforts have been focused on statins.

Statins are medications that have been widely used to treat hypercholesterolemia in patients at high risk of developing atherosclerosis and having episodes of cardiovascular disease. Statins are powerful cholesterol-lowering agents that inhibit cholesterol biosynthesis in the liver and consequently have demonstrated beneficial effects in the primary and secondary prevention of ischemic heart disease. These drugs inhibit the enzyme 3-hydroxy-3-methylglutaryl Coenzyme A reductase (HMG CoA reductase) (Essig, M. et al, 3-hydroxi-3-methylglutaryl coenzyme A reductase inhibitors increase fibrinolytic activity in rat aortic endothelial cells. Circ. Res. 1998;83:683-690), responsible for the conversion in the liver of HMG-CoA to mevalonic acid, cholesterol precursor, resulting in a reduction in low-density cholesterol (LDL) levels and other changes in the lipid profile.

Based on the background described above, concerning the inflammatory mechanisms involved in the development of cardiovascular disease, there is a certain parallelism between these and the inflammatory changes responsible for the pathogenesis of periodontal disease. A growing interest in studying the potential relationship between both diseases has been developed in recent decades, suggesting the existence of common underlying mechanisms that would link periodontal and cardiovascular health. In vitro studies and in vivo trials have suggested the potential use of statins in periodontal disease; even a few retrospective studies show that patients with advanced chronic periodontitis treated with statins improve their outcomes. Finally, it has been described that statins also have effects on increasing bone formation, increasing angiogenesis, and bactericidal effects. In the context of the use of statins in dental compositions, US patent N°6300377 discloses a liquid composition comprising Coenzyme Q. The disclosed composition is intended for treating periodontitis and gum-related disorders. This composition can be formulated in mouthwashes, toothpaste, or chewing gum. Among the components that the composition may comprise are gelling agents, surfactants, triglycerides, and phospholipids. Furthermore, it is mentioned that it may optionally comprise a second active agent, which may be a statin, such as lovastatin, simvastatin, atorvastatin, fluvastatin, pravastatin, mevastatin, cerivastatin, in a concentration between 0.01% to 20% in relation to the total weight of the composition.

Patent application WO2014030132 discloses the use of a topical composition comprising atorvastatin in a range between 0.2% to 10% as the only active ingredient for the manufacture of a composition useful in the primary prevention or the treatment of human or animal periodontal disease.

The publication "*the use of topical subgingival application of simvastatin gel in treatment of peri implant mucositis: A pilot study*", corresponds to a publication about the treatment of peri-implant mucositis through the topical use of simvastatin. Simvastatin is formulated in a gel with a concentration of 1.2%. The document also discloses the use of oral antiseptics for peri-implant mucositis, including chlorhexidine in a dose of 0.12% and 0.2%, which is applied in the form of a gel. However, this publication does not mention the use of this composition for the treatment of periodontitis.

Finally, the publication from Kamińska et al, 2018: "Effects of statins on multispecies oral biofilm identify simvastatin as a drug candidate targeting Porphyromonas gingivalis*"* discloses research about the antimicrobial activity of statins against periodontal homeostasis bacteria that cause chronic periodontitis, such as *Fusobacterium nucleatum*, *Porphyromonas gingivalis* and *Tanerella forsythia.* This study investigated the effectiveness of four types of statins (atorvastatin, fluvastatin, lovastatin, and simvastatin) on these bacteria, which showed that the effectiveness only occurs in some of the bacteria studied and that, in addition, said effectiveness is very dependent on the concentration of each of the statins. This is demonstrated when the statins atorvastatin and simvastatin were not able to produce any bactericidal effect. For example, on *Fusobacterium nucleatum*, despite its use at high concentrations.

Consequently, despite the research and efforts made to combat periodontal disease, it has not yet been possible to find an effective solution for this disease.

An objective of the present invention is to provide an effective alternative in the treatment and prevention of gingivitis, periodontitis, especially chronic and aggressive periodontitis.

In response to this need, the present invention provides a pharmaceutical composition comprising a combination of statins with antiseptic and/or antibacterial agents, together with auxiliary facilitating agents, which produce a synergistic antibacterial effect to combat different species of bacteria associated with the appearance of chronic and aggressive periodontal disease. This synergistic composition allows the use of a wide range of statins and different types, which are shown to be effective even at very low concentrations.

This surprising finding allows us to present a new therapy to control this pathology, which aims to treat bacterial infection, one of the main factors in the etiology of periodontitis.

### SUMMARY OF THE INVENTION

The present invention describes pharmaceutical compositions comprising at least one statin in combination with at least one oxidizing antiseptic and/or antibacterial agent and at least one pharmaceutically acceptable auxiliary facilitating agent.

The compositions according to the present invention are formulated, for example, but not limited to toothpaste, mouthwash, dental floss, tablets to dissolve in the mouth, controlled release intraoral elements or devices, among others.

These compositions have applications in the treatment of diseases of the soft tissues present in the oral cavity.

### BRIEF DESCRIPTION OF THE FIGURES

- Figure 1:: Photographs of periodontal disease progression.
- Figure 2:: Photographs of plates with a lawn of bacteria *Fusobacterium nucleatum* and compositions 1, 2, 3 and 4.
- Figure 3:: Photograph of serial dilutions and their controls for Composition 1.
- Figure 4:: Photograph of serial dilutions and their controls for Composition 1.
- Figure 5:: Photographs of plates with control solution and Composition 1.
- Figure 6:: Photograph of serial dilutions and their controls for Composition 2.
- Figure 7:: Photograph of serial dilutions and their controls for Composition 2.
- Figure 8:: Photographs of plates with control solution and Composition 2.
- Figure 9:: Photograph of serial dilutions and their controls for Composition 3.
- Figure 10:: Photograph of serial dilutions and their controls for Composition 3.
- Figure 11:: Photographs of plates with control solution and Composition 3.
- Figure 12:: Photograph of serial dilutions and their controls for Composition 4.
- Figure 13:: Photograph of serial dilutions and their controls for Composition 4.
- Figure 14:: Photographs of plates with control solution and Composition 4.
- Figure 15:: Photographs of plates with control solution and Composition 1.
- Figure 16:: Photographs of plates with control solution and Composition 2.
- Figure 17:: Photographs of plates with control solution and Composition 3.
- Figure 18:: Photographs of plates with control solution and Composition 4.
- Figure 19:: Photographs of plates with a lawn of bacteria *Aggregatibacter actinomycetemcomitans* and compositions 1, 2, 3 and 4.
- Figure 20:: Photograph of serial dilutions and their controls for Composition 1.
- Figure 21:: Photograph of serial dilutions and their controls for Composition 1.
- Figure 22:: Photographs of plates with control solution and Composition 1.
- Figure 23:: Photograph of serial dilutions and their controls for Composition 2.
- Figure 24:: Photograph of serial dilutions and their controls for Composition 2.
- Figure 25:: Photographs of plates with control solution and Composition 2.
- Figure 26:: Photograph of serial dilutions and their controls for Composition 3.
- Figure 27:: Photograph of serial dilutions and their controls for Composition 3.
- Figure 28:: Photographs of plates with control solution and Composition 3.
- Figure 29:: Photograph of serial dilutions and their controls for Composition 4.
- Figure 30:: Photograph of serial dilutions and their controls for Composition 4.
- Figure 31:: Photographs of plates with control solution and Composition 4.
- Figure 32:: Photographs of plates with control solution and Composition 1.
- Figure 33:: Photographs of plates with control solution and Composition 2.
- Figure 34:: Photographs of plates with control solution and Composition 3.
- Figure 35:: Photographs of plates with control solution and Composition 4.
- Figure 36:: Photographs of plates with a lawn of bacteria *Fusobacterium nucleatum* and Compositions 7, 8, 9, 10 and 11.
- Figure 37:: Photograph of serial dilutions and their controls for Composition 7.
- Figure 38:: Photographs of plates with control solution and Composition 7.
- Figure 39:: Photograph of serial dilutions and their controls for Composition 10.
- Figure 40:: Photographs of plates with control solution and Composition 10.
- Figure 41:: Photograph of serial dilutions and their controls for Composition 7.
- Figure 42:: Photograph of serial dilutions and their controls for Composition 10.
- Figure 43:: Photographs of plates with a lawn of bacteria *Aggregatibacter actinomycetemcomitans* and Compositions 7, 8, 9, 10, and 11.
- Figure 44:: Photograph of serial dilutions and their controls for Composition 7.
- Figure 45:: Photograph of serial dilutions and their controls for Composition 7.
- Figure 46:: Photograph of serial dilutions and their controls for Composition 9.
- Figure 47:: Photograph of serial dilutions and their controls for Composition 9.
- Figure 48:: Photograph of serial dilutions and their controls for Composition 7.
- Figure 49:: Photograph of serial dilutions and their controls for Composition 9.
- Figure 50:: Photographs of plates with a lawn of bacteria *Fusobacterium nucleatum* and Compositions A, S, R, and P.
- Figure 51:: Photographs of plates with a lawn of bacteria *Aggregatibacter actinomycetemcomitans* and Compositions A, S, R, and P.

### DETAILED DESCRIPTION OF THE INVENTION

The present invention relates to a pharmaceutical composition comprising at least one statin, at least one antiseptic and/or antibacterial agent with oxidizing properties, and at least one pharmaceutically acceptable auxiliary facilitating agent. Likewise, the present invention is directed to the use of a pharmaceutical composition to prevent and/or treat diseases of the soft and hard tissues surrounding the tooth present in the oral cavity. Specifically, to prevent and/or treat periodontal disease.

The compositions of the present invention comprise at least one statin selected from, but not limited to lovastatin, rosuvastatin, pravastatin, simvastatin, fluvastatin, cerivastatin, atorvastatin, and pitavastatin or pharmaceutically acceptable salts thereof.

The compositions of the present invention also comprise at least one oxidizing antiseptic and/or antibacterial agent selected from, but not limited to hydrogen peroxide, salts, or oxysalts containing chlorine and/or bromine, such as sodium hypochlorite, sodium chlorite, among others. The compositions of the present invention also comprise at least one pharmaceutically acceptable auxiliary facilitating agent selected from, but not limited to at least one essential oil, at least one phenol-derived preservative agent, and at least one wetting agent.

The preservative or preservative auxiliary agent of the present invention refers to compounds that help delay or prevent deterioration of the composition. Examples of phenolic preservative agents that can be used according to the invention include phenol, parachloromethoxylenol, parachlorophenol, cresol, hexylresorcinol, alkylparabens such as methylparaben, ethylparaben, propylparaben, butylparaben, isopropylparaben, and isobutylparaben.

The auxiliary wetting agent of the present invention can moisturize the oral cavity and, in addition, promote and stimulate salivation and prevent its dehydration, as well as solubilize certain active ingredients. Examples of wetting agents, according to the invention, include xylitol, glycerin, sorbitol, and glycols, such as propylene glycol and polyethylene glycol and mixtures thereof. The auxiliary facilitating agent of the present invention is also selected from at least one essential oil selected from eugenol, camphor, thymol (also referred to as eucalyptol), and menthol, among other agents widely used in dentistry, which have organoleptic properties that improve the patient adherence to therapy.

The pH of the oral compositions of the invention is in the range of 6.0 to 8.0, preferably between 6.5 to 7.5. Therefore, the compositions of the present invention may optionally comprise an auxiliary pH-regulating agent (buffers) selected from bicarbonate and phosphate, among others. Optionally, the composition comprises at least one plant extract and a flavoring, or combinations thereof.

The composition of the invention may also comprise other agents that may provide benefits to the patient, such as antibiotics, zinc salts and/or fluoride derivatives.

Optionally, the composition of the invention also comprises flavoring agents which can be selected from any compound or mixture thereof that allows the flavor of the composition to be enhanced.

According to the invention, appropriate flavorings are ingredients that provide a fragrance and/or other sensory effect in the mouth, such as a cooling or warming sensation. Such ingredients include, but are not limited to, menthol, isoamylacetate, methyl acetate, methylsalicylate, methyl-lactate, camphor, eucalyptus oil, eucalyptol, oxanone, orange essence, cherry essence, peppermint oil, anise oil, anethole vanillin, papaya, and ethanol, among others.

The composition of the invention may further comprise sweetening agents. These sweetening agents for use in oral compositions include, for example, saccharin, dextrose, sucrose, sucralose, stevia, allulose, and tagatose, among others.

The compositions of the invention can be prepared by mixing each ingredient and incorporating them in an appropriate amount of a pharmaceutically acceptable excipient, such as a vehicle, solvent, and others.

The compositions according to the invention further comprise one or more pharmaceutically acceptable excipients, which, at the concentration used, do not cause any undesirable effects in the subjects to whom they are administered. Such pharmaceutically acceptable excipients are well known in the art. They can be selected from the group consisting of lipophilic or hydrophilic carriers, diluents (such as water, alcohol, lactose, maltose, and trehalose), binders, disintegrating agents, flow-improving agents, pH adjusting agents, stabilizing agents, viscosity adjustment agents, preservatives, gelling or swelling agents, surfactants, suspending agents. As the skilled person recognizes, the specific choice of pharmaceutically acceptable excipients depends on the specific form or formulation.

The person skilled in the art will understand that the total of all the ingredients (components) used in the composition of the invention add up to 100% by weight of the total composition. Furthermore, unless otherwise indicated, all percentages described herein refer to percentages by weight of the total composition.

In a preferred embodiment, the compositions of the present invention are formulated topically or orally, for example, but not limited to toothpaste, mouthwash, oral dissolving tablets, elements or devices for intraoral use, and dental floss.

In another embodiment, the present invention relates to a pharmaceutical kit comprising the previously described pharmaceutical composition and the corresponding instructions for use.

In another embodiment, the compositions of the present invention are used in the primary prevention and treatment of periodontal disease.

In a further embodiment, the present invention relates to a treatment method to prevent and/or treat diseases of the soft tissues present in the oral cavity, specifically, to prevent and/or treat periodontal disease, which comprises the administration of the compositions of the present invention.

### MODALIDADES PREFERIDAS DE LA INVENCIÓN

The present invention is directed to an oral pharmaceutical composition comprising at least one statin and at least one antiseptic and/or antibacterial agent.

In one embodiment of the invention, the composition further comprises at least one pharmaceutically acceptable auxiliary facilitating agent.

In a preferred embodiment of the invention, the at least one antiseptic and/or antibacterial agent of the composition is an oxidizing compound, which is selected from hydrogen peroxide and sodium hypochlorite, preferably hydrogen peroxide, which is present in an amount of at least less 0.0014% by weight with respect to the total composition.

In another embodiment of the invention, sodium hypochlorite is present in an amount of at least 0.0023% by weight with respect to the total composition.

In one embodiment of the invention, the at least one statin is fat-soluble, which is selected from lovastatin, simvastatin, atorvastatin, and pitavastatin.

In another embodiment of the invention the statin is water-soluble, which is selected from pravastatin, fluvastatin, and rosuvastatin.

In a preferred embodiment of the invention, the statin is present in an amount of at least 0.00029% by weight with respect to the total composition.

In one embodiment of the invention, the at least one auxiliary facilitating agent is a preservative derived from phenol, which is selected from phenol, parachlorophenol, alkylparaben and a mixture thereof.

In a preferred embodiment of the invention, parachlorophenol is present at least 0.00006% by weight relative to the composition's total. Wherein the parachlorophenol includes a vehicle corresponding to camphor in an amount of at least 0.00009% by weight relative to the total of the composition.

In another embodiment of the invention, the alkylaparaben is selected from methylparaben, ethylparaben, propylparaben, butylparaben, isopropylparaben, and isobutylparaben, preferably methylparaben, which is present in an amount of at least 0.00071% by weight relative to the total of the composition.

In an embodiment of the invention, the at least one auxiliary facilitating agent in an essential oil, preferably eugenol, camphor, or a combination thereof.

In a preferred embodiment of the invention eugenol is present in an amount of at least 0.00014% by weight relative to the total of the composition.

Even in another embodiment of the invention the at least one auxiliary facilitating agent is a wetting agent, preferably corresponding to xylitol, which is present in an amount of at least 0.04% by weight relative to the composition's total.

In an embodiment of the invention, the at least one auxiliary facilitating agent is a diluent, preferably purified water.

In an embodiment of the invention, the composition further comprises a pH regulator (buffer), preferably a bicarbonate buffer.

In another embodiment of the invention, the composition further comprises flavoring agents, sweeteners, or a mixture thereof.

Furthermore, the present invention is directed to a pharmaceutical formulation comprising the composition according to the present invention wherein said formulation is selected from toothpaste, gel, mouthwash, dental floss, tablets to dissolve in the mouth, and intraoral controlled-release elements or devices such as microparticle or nanoparticle capsules.

Additionally, the present invention is directed to the use of the composition to manufacture a medication to prevent and/or treat diseases of the soft and hard tissues surrounding the tooth present in the oral cavity. Preferably, the disease of the soft and hard tissues surrounding the tooth is selected from gingivitis and periodontitis. Even more preferably, chronic periodontitis and severe periodontitis.

The present invention is also directed to a pharmaceutical kit for oral use comprising the composition according to claim 1 and instructions for use.

### APPLICATION EXAMPLES

### EXAMPLE 1

Table 1 shows the different prepared compositions representative of the invention.

**Table 1**

| **Composition 1 (% by weight)** | **Composition 2 (% by weight)** | **Composition 3 (% by weight)** | **Composition 4 (% by weight)** | **Composition 5 (% by weight)** | **Composition 6 (% by weight)** |
|---|---|---|---|---|---|
| Atorvastatin (0.02) | Simvastatin (0.02) | Rosuvastatin (0.02) | Pravastatin (0.02) | Pravastatin (0.02) | Rosuvastatin (0.1) |
| H₂O₂ (0.1) | H₂O₂ (0.1) | H₂O₂ (0.1) | H₂O₂ (0.1) | H₂O₂ (0.1) | H₂O₂ (0.1) |
| Parachlorophenol (0.004) | Parachlorophenol (0.004) | Parachlorophenol (0.004) | Parachlorophenol (0.004) | Methylparaben (0.1) | Methylparaben (0.05) |
| Eugenol (0.01) | Eugenol (0.01) | Eugenol (0.01) | Eugenol (0.01) | Eugenol (0.01) | Eugenol (0.01) |
| Xylitol (3.0) | Xylitol (3.0) | Xylitol (3.0) | Xylitol (3.0) | Xylitol (3.0) | Xylitol (3.0) |
| Camphor (0.006) | Camphor (0.006) | Camphor (0.006) | Camphor (0.006) | Camphor (0.006) | Camphor (0.006) |

Additionally, compositions from Table 1 comprise flavoring agents, sweeteners, a buffer agent, and purified water.

### EXAMPLE 2

To evaluate the antimicrobial activity of the compositions of the invention against bacteria associated with chronic and aggressive periodontitis, 4 compositions were tested with the active ingredients indicated in Table 1 for Compositions 1 to 4, using water as diluent until completing the 100% by weight, on the bacteria *Fusobacterium nucleatum* and *Aggregatibacter actinomycetemcomitans.*

The inhibitory activity of the compositions was studied using the minimum inhibitory concentration (MIC) and the minimum bactericidal concentration (MBC).

### Methodology

### Determination of inhibitory activity

The standardized Kirby-Bauer disk diffusion protocol was used to carry out this study. To establish whether the composition has inhibitory activity on the bacteria studied, *Aggregatibacter actinomycetemcomitans* and *Fusobacterium nucleatum,* the loading of sensidiscs was carried out with 5 µL, 10 µL and 20 µL of each composition, after incubation at room temperature for 8 h they were tested on a lawn of the bacteria studied in TSVB medium, incubating the plates for 48 h at 37°C in capnophilia.

### Determination of minimum inhibitory concentration (MIC)

In this study, it must first be determined which compositions and/or compounds present inhibitory activity, and the following dilutions of the compositions described in Table 1 are carried out: 1/10, 1/50, 1/60, 1/70, 1/80, 1/90, 1/100, 1/500, 1/1000 and 1/2000 in 2 mL of liquid BHI medium, to which an isolated colony of the target bacteria is added, and allowed to incubate in capnophilia at 37° C for 24 hours. Subsequently, selected samples are counted through 8 serial dilutions (1:100) in a multiwell plate; 5 µL of each dilution are seeded in triplicate on a BHI plate and incubated in anaerobiosis for 24 h.

### Determination of the minimum bactericidal concentration (MBC)

This study is carried out by seeding an isolated colony of the strain in 8 test tubes with 2 mL of liquid BHI medium; these tubes are incubated for 24 h in capnophilia, and then adding the composition or compound to be evaluated in the ratio 1/10, 1/50, 1/100, 1/500, 1/1000 and 1/2000. After a new 24-h incubation, the selected samples are counted through 8 serial dilutions (1:100) in a multiwell plate. 5 µL of each dilution are seeded in triplicate on a BHI plate and incubated in capnophilia for 24 h.

### Assay 2.1: Evaluation of antimicrobial activity on Fusobacterium nucleatum

The preliminary study of the compounds was based on determining whether the compounds have inhibitory activity. Through this experimentation, it was possible to establish that Compositions 1, 2, 3, and 4 have inhibitory activity against *Fusobacterium nucleatum.*

In Figure 2, you can see plates with a lawn of the bacteria and a sensidisc previously loaded with 10 µL and 20 µL of each composition placed over the culture.

### Determination of Minimum Inhibitory Concentration (MIC)

### Composition 1

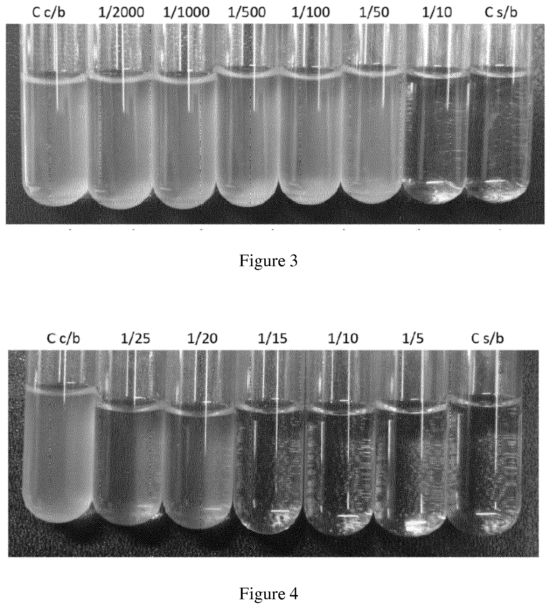

Serial dilutions from 1/10 to 1/2000 were prepared with their respective controls, with and without bacteria. A preliminary study established the MIC within 1/50 and 1/10 dilutions (Figure 3). The subsequent study, located in said preliminary dilution range, showed a variation in growth between 1/20 and 1/15, with growth being non-existent in the latter (Figure 4).

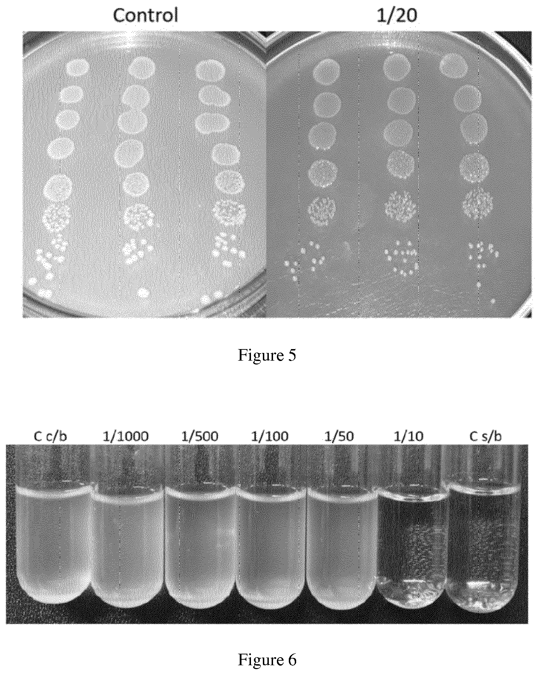

The count of the control and 1/20 solutions (Figure 5) showed us that there is a clear decrease in growth, finding 9.43×10⁹ UFC/mL and 2.86×10⁹ UFC/mL, respectively, which establish a 69,9% inhibition for the 1/20 dilution of Composition 1.

### Composition 2

Serial dilutions from 1/10 to 1/2000 of this composition were prepared with their respective controls, with and without bacteria, to allow for establishing the Minimum Inhibitory Concentration.

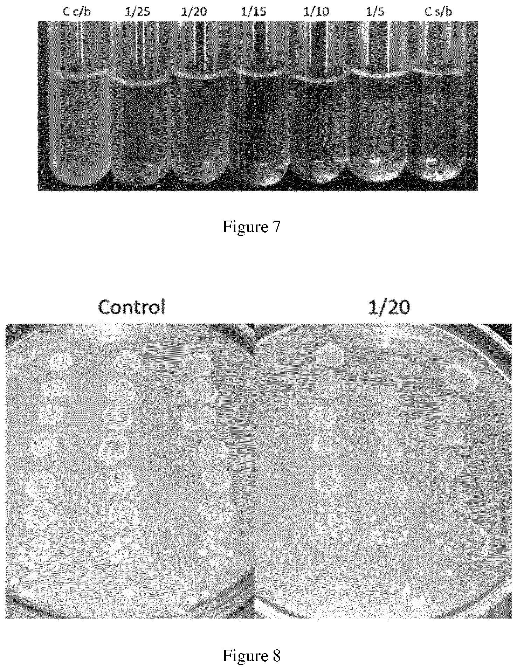

A preliminary study established the MIC within 1/50 and 1/10 dilutions (Figure 6), and the subsequent study of dilutions within said range showed a variation in growth between 1/20 and 1115 (Figure 7), with growth being non-existent in the latter.

The count of the control and 1/20 solutions (Figure 8) showed that there is a clear decrease in growth, finding 9.43×10⁹ UFC/mL and 4.6×10⁹ UFC/mL, respectively, which results in an inhibition of 85,31% for the 1/20 dilution of Composition 2.

### Composition 3

Serial dilutions from 1/10 to 1/2000 of this composition were prepared with their respective controls, with and without bacteria, and then a second group of dilutions from 1/5 to 1/25 with their respective controls with and without bacteria to allow establishing the Minimum Inhibitory Concentration.

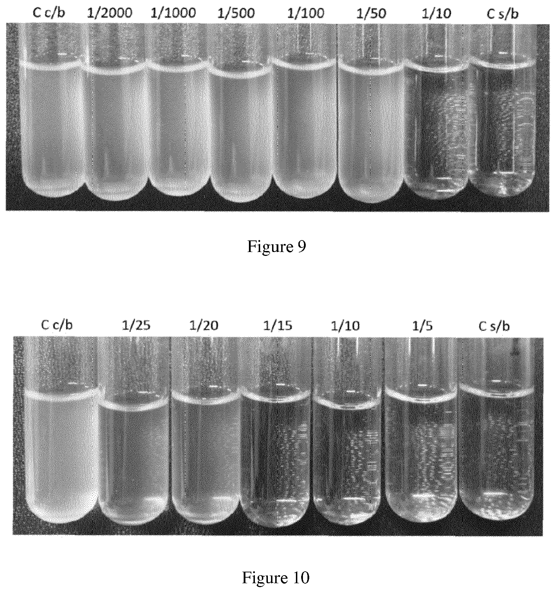

The preliminary study established the MIC within dilutions 1/50 and 1/10 (Figure 9), and the subsequent study of dilutions within that range (Figure 10) showed a variation in growth between 1/20 and 1/15, with non-existent growth in the latter.

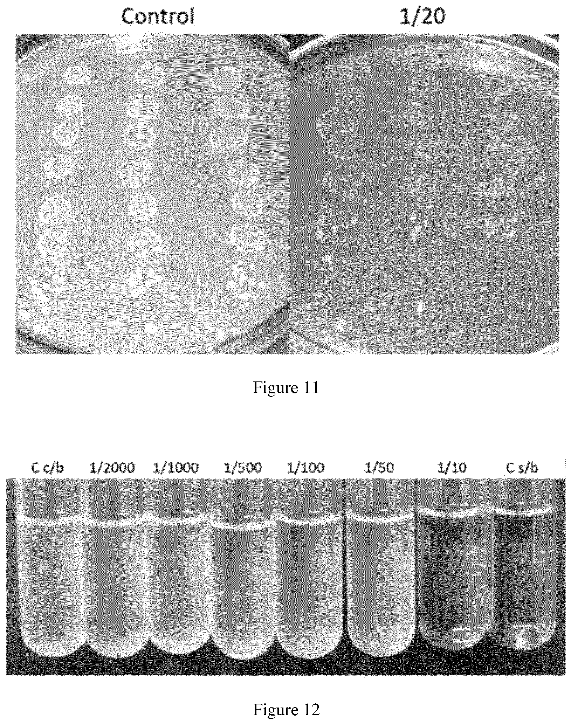

The count of the control and 1/20 solutions (Figure 11) showed that there is a clear decrease in growth, finding 9.43×10⁹ UFC/mL and 1.4×10⁹ UFC/mL, respectively, which establish an inhibition of 51.73% for the 1/20 dilution of Composition 3.

### Composition 4

Serial dilutions from 1/10 to 1/2000 of this composition were prepared with their respective controls, with and without bacteria, and then a second group of dilutions from 1/5 to 1/25 with their respective controls with and without bacteria, to allow for establishing the Minimum Inhibitory Concentration.

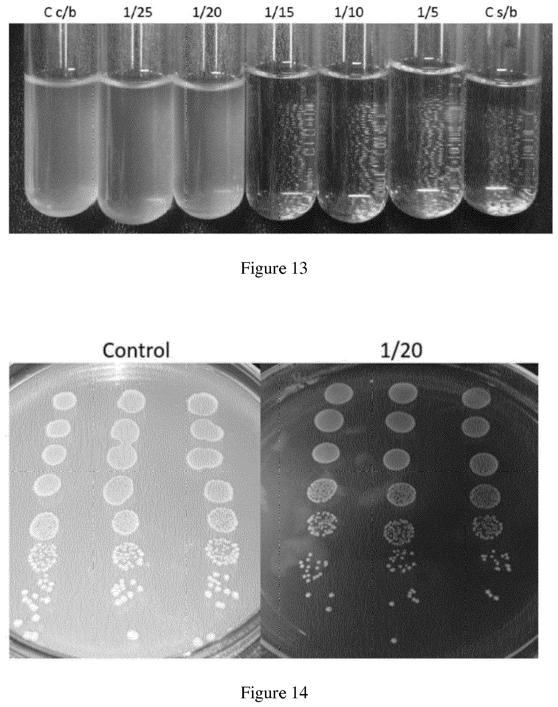

The preliminary assay established the MIC within dilutions 1/50 and 1/10 (Figure 12), and the subsequent study of dilutions within said range (Figure 13) showed a variation in growth between 1/20 and 1/15, with non-existent growth in the latter.

The count of the control and 1/20 solutions (Figure 14) showed that there is a clear decrease in growth, finding 9.43×10⁹ UFC/mL and 3.4×10⁹ UFC/mL, respectively, which established an inhibition of 64.33% for the 1/20 dilution of the Composition 4.

### Determination of the Minimum Bactericidal Concentration (MBC)

### Composition 1

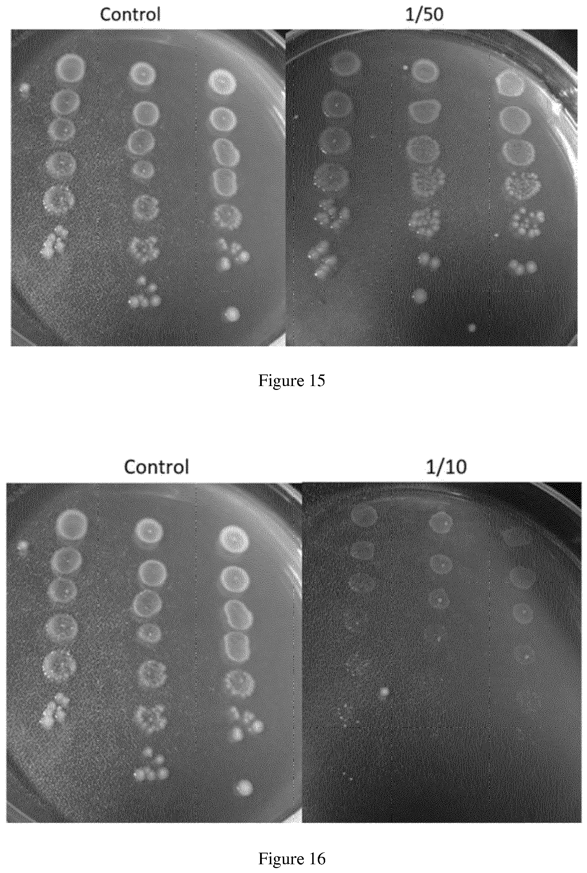

Counting the dilutions (Figure 15) showed that the bacteria were dead in the 1/10 dilution of Composition 1, establishing the MBC at the 1/50 dilution. 4.4×10⁹ CFU/mL were found in the control and 13.6×10⁸ CFU/mL in the 1/50 dilution, which established a bactericidal action of 91.82% for the 1/50 dilution of Composition 1.

### Composition 2

Counting the dilutions (Figure 16) established the MBC in the fraction 1/10, finding 4.4×10⁹ UFC/mL in the control and 5.2×10⁹ UFC/mL in the 1/10 fraction, which allowed to establish a bactericidal action of 88.2% for the 1/10 dilution of Composition 2.

### Composition 3

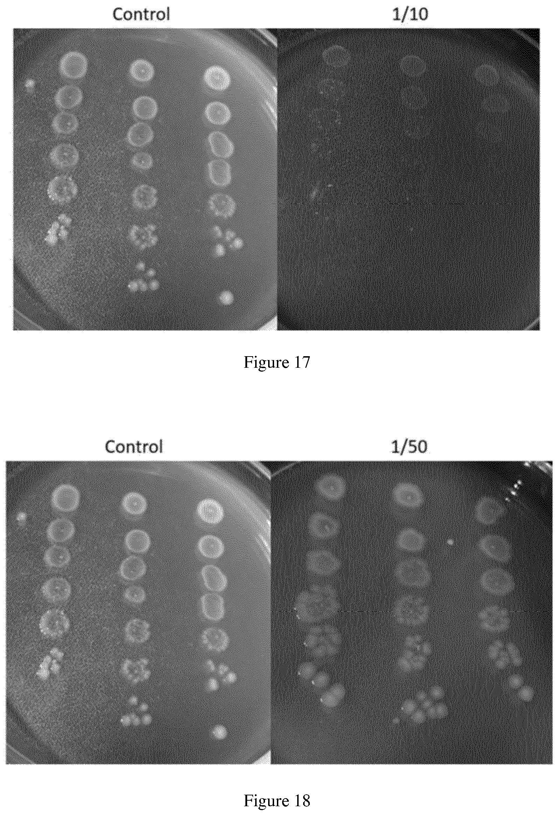

Serial dilutions from 1/10 to 1/2000 allowed the MBC to be determined by counting the dilutions (Figure 17), establishing the MBC at the 1/10 dilution, finding 4.4×10⁹ UFC/mL in the control and 2.8×10⁹ UFC/mL in the 1/10 fraction, which results in a bactericidal action of 36.4% for the 1/10 dilution of Composition 3.

### Composition 4

Serial dilutions from 1/10 to 1/2000 allowed the MBC to be determined by counting the dilutions (Figure 18), showing that at the 1/10 dilution, the bacteria were dead, establishing the MBC in the 1/50 fraction, finding 4,4×10⁹ UFC/mL in the control and 4.1×10⁸ UFC/mL in the 1/50 fraction, which established a bactericidal action of 90.91% for the 1/50 dilution of Composition 4.

### Conclusions

Compositions 1, 2, 3, and 4 generated an inhibitory effect on *Fusobacterium nucleatum.*

Table 2 shows the effectiveness of the dilutions of each of the compositions of the present invention in inhibiting the growth of *Fusobacterium nucleatum.*

**Table 2**

| | % of inhibition |
|---|---|
| Composition 1 (1/20 dilution) | 69.9 |
| Composition 2 (1/20 dilution) | 85.3 |
| Composition 3 (1/20 dilution) | 51.7 |
| Composition 4 (1/20 dilution) | 64.3 |

For compositions 1, 2, 3, and 4, the 1/10 dilution achieved an inhibition of 100%.

Table 3 shows the effectiveness of the dilutions of each of the compositions of the present invention in eliminating *Fusobacterium nucleatum.*

**Table 3**

| | % of elimination |
|---|---|
| Composition 1 (1/50 dilution) | 91.8 |
| Composition 2 (1/10 dilution) | 88.2 |
| Composition 3 (1/50 dilution) | 36.4 |
| Composition 4 (1/50 dilution) | 90.9 |

For all the compositions according to the present invention, the 1/10 dilution achieved an elimination of 100% of the bacteria *Fusobacterium nucleatum.*

It is interesting to note that in some compositions, a better bactericidal than inhibitory effect is observed. For example, in Composition 1, a dilution factor of 20 allows an inhibition of 69.9% of *Fusobacterium nucleatum,* while a higher dilution, FD 50, showed an elimination of 91.8% of the same bacteria. The inventors believe that this behavior is because a bioactive component at high concentrations may aggregate (agglomerate) and decrease its activity, and, therefore, when diluted, its activity increases because it disaggregates. In the example of Composition 1 containing atorvastatin, this explanation is very coherent since said statin has low water solubility, so when diluted, a total dispersion is achieved in the final solution, reaching higher activity levels.

### Assay 2.2: Evaluation of the antimicrobial activity on Aggregatibacter actinomycetemcomitans

The preliminary study of the compounds was based on establishing whether the compounds have inhibitory activity. This experimentation established that compositions 1, 2, 3, and 4 present inhibitory activity against *Aggregatibacter actinomycetemcomitans.*

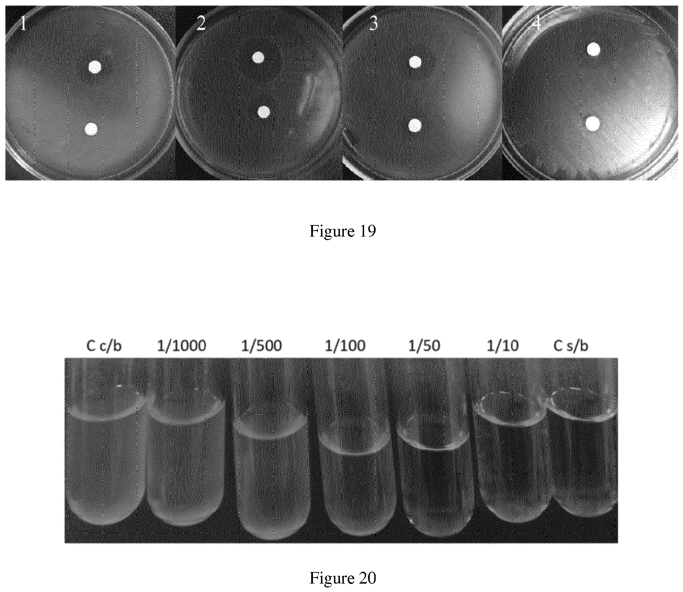

In Figure 19 plates with a lawn of the bacteria can be observed, over the culture a sensidisc previously loaded with 10 µL and 20 µL of each composition was placed..

### Determination of Minimum Inhibitory Concentration (MIC)

### Composition 1

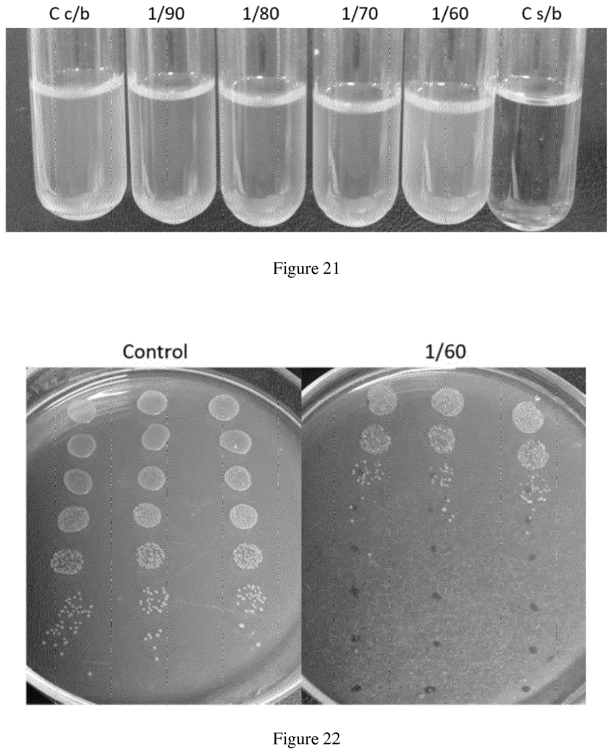

The serial dilutions of this composition allowed for the establishment of the minimum inhibitory concentration. The preliminary study established the MIC within dilutions 1/100 and 1/50 (Figure 20), and the subsequent study of dilutions within this range (Figure 21) showed growth up to fraction 1/60, having the fraction 1 /50 of Composition 1 non-existent growth.

Counting of control and 1/60 solutions (Figure 22) showed that there is a clear decrease in growth, finding 5.7×10⁹ UFC/mL for the control and 5.46×10⁶ UFC/mL for the 1/60 dilution of Composition 1, which establishes an inhibition of 99.91% for the 1/60 dilution.

### Composition 2

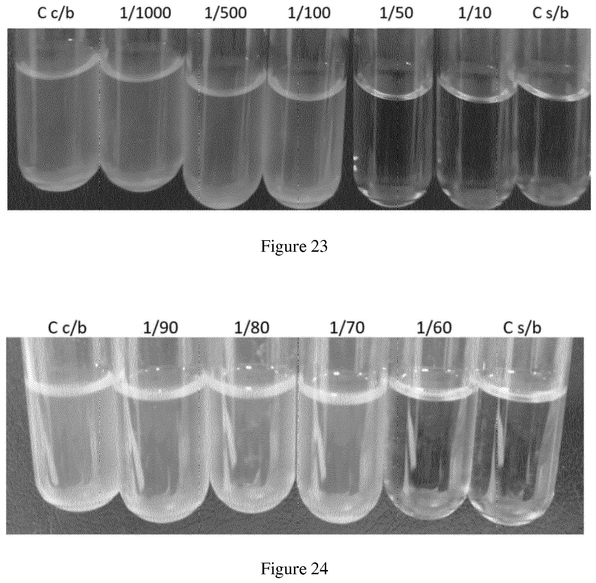

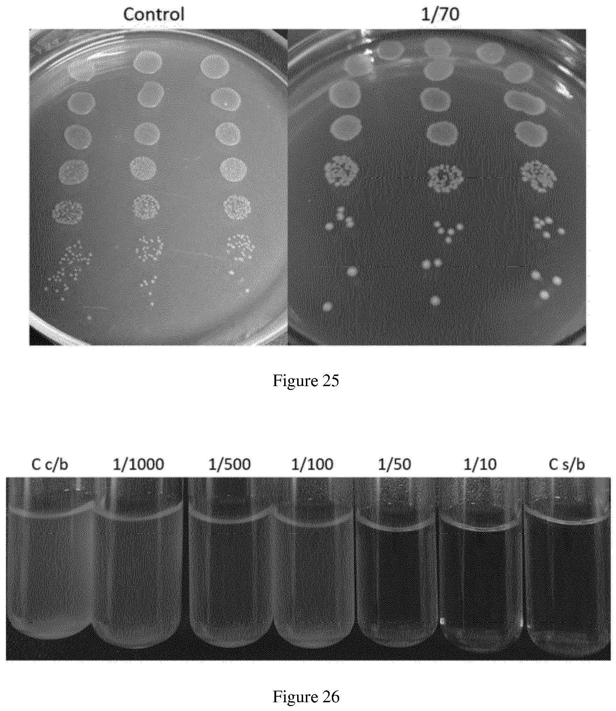

The preliminary study of serial dilutions established the MIC within the 1/100 and 1/50 dilutions (Figure 23), and the subsequent study of dilutions within said range (Figure 24) showed growth up to the 1/70 fraction, having the 1/60 fraction of Composition 1 non-existent growth. Counting of control and 1/70 dilution of Composition 2 (Figure 25) showed that there is a clear decrease in growth, finding 5.7×10⁹ UFC/mL and 1.106×10⁹ UFC/mL, respectively, which established an inhibition of 80,6% for the 1/70 dilution of Composition 2.

### Composition 3

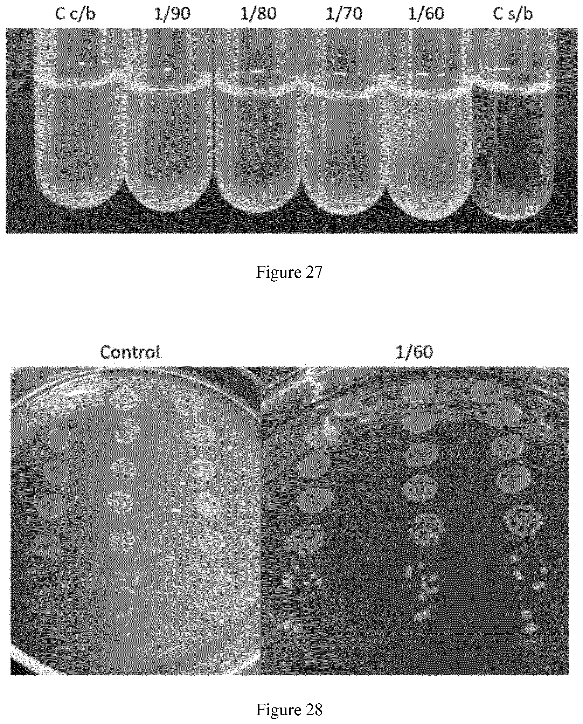

The preliminary assay established the MIC within the 1/100 and 1/50 dilutions (Figure 26), and the subsequent study of dilutions within said range (Figure 27) showed growth up to the 1/60 fraction, having the 1/50 fraction of Composition 3 non-existent growth.

Counting control and 1/60 dilutions (Figure 28) showed that there is a clear decrease in bacterial growth, finding 5.7×10⁹ UFC/mL and 9.52×10⁸ UFC/mL, respectively, which showed an inhibition of 83,3% for the 1/60 dilution of Composition 3.

### Composition 4

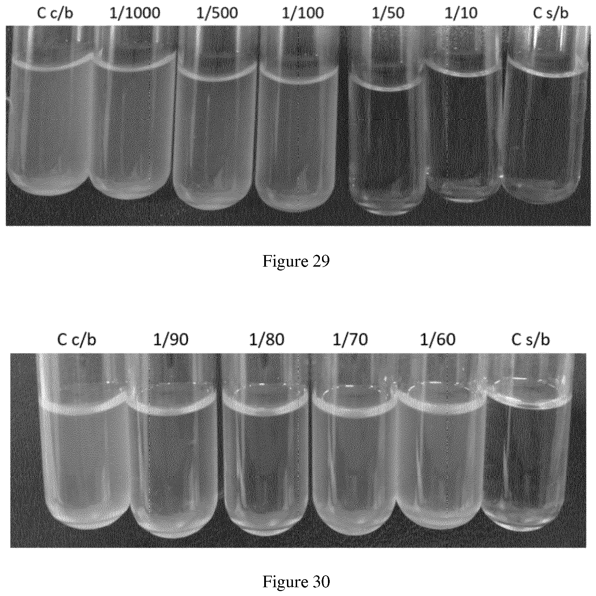

The preliminary assay established the MIC within the 1/100 and 1/50 dilutions (Figure 29), and the subsequent study of dilutions within said range (Figure 30) showed growth up to the 1/60 fraction, having the 1/50 fraction of Composition 4 a non-existent growth.

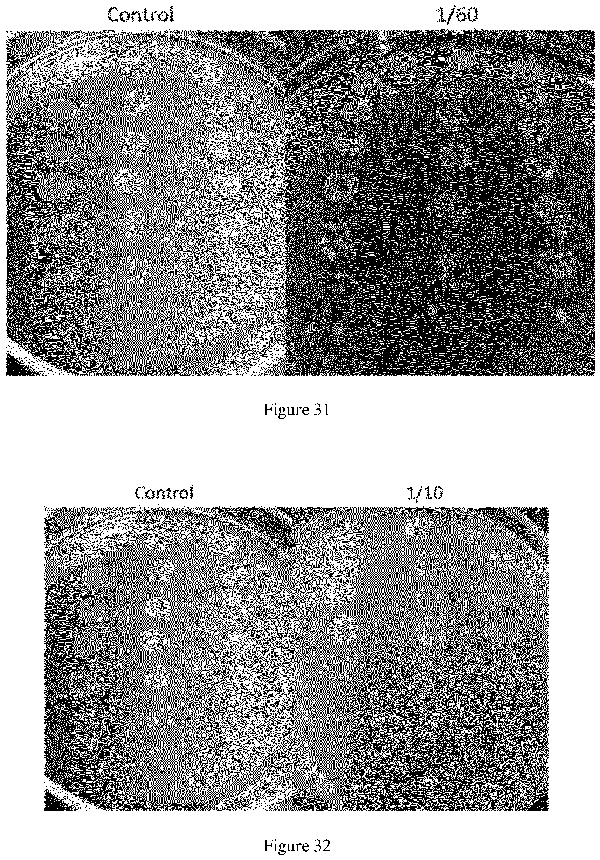

Counting the control and 1/60 solutions (Figure 31) showed that there is a clear decrease in growth, finding 5.7×10⁹ UFC/mL and 9.8×10⁸ UFC/mL, respectively, which established an inhibition of 82,81% for the 1/60 dilution.

### Determination of the Minimum Bactericidal Concentration (MBC)

### Composition 1

Serial dilutions from 1/10 to 1/2000 allowed the MBC to be established by counting the dilutions (Figure 32), establishing the MBC at the 1/10 fraction, finding 4.4×10⁹ UFC/mL in the control and 5.12×10⁸ UFC/mL in the 1/10 fraction, which established a bactericidal action of 91.08% for the 1/10 dilution.

### Composition 2

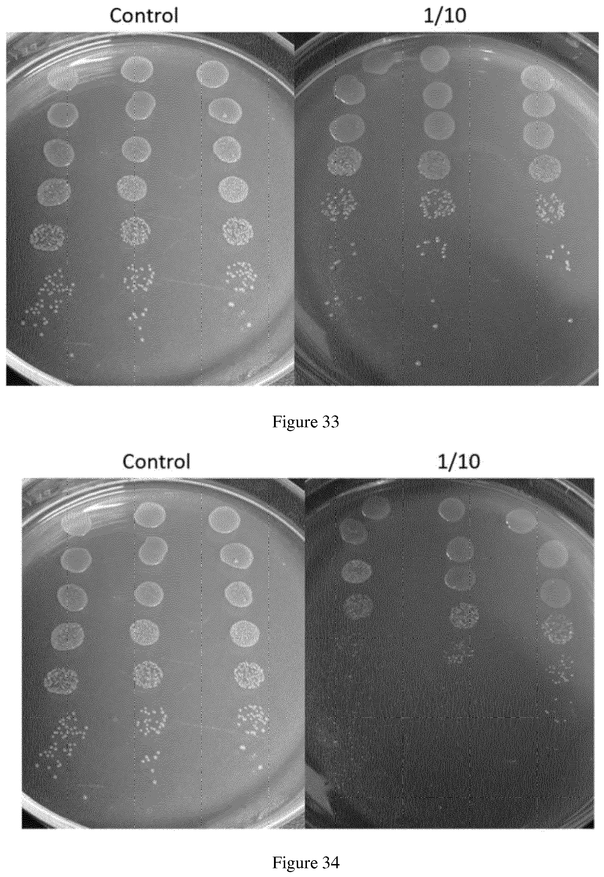

Serial dilutions from 1/10 to 1/2000 allowed the MBC to be established by counting the dilutions (Figure 33), which was established at the 1/10 fraction. 4.4×10⁹ UFC/mL were found in the control and 8.4×10⁸ UFC/mL were found in the 1/10 fraction, which determined a bactericidal action of 85.26% for the 1/10 dilution of Composition 2.

### Composition 3

Serial dilutions from 1/10 to 1/2000 allowed the MBC to be established by counting the dilutions (Figure 34), which was established at the 1/10 fraction. 4.4×10⁹ UFC/mL were found in the control and 4.8×10⁸ UFC/mL were found in the 1/10 fraction, which resulted in a bactericidal action of 91,57% for the 1/10 dilution of Composition 3.

### Composition 4

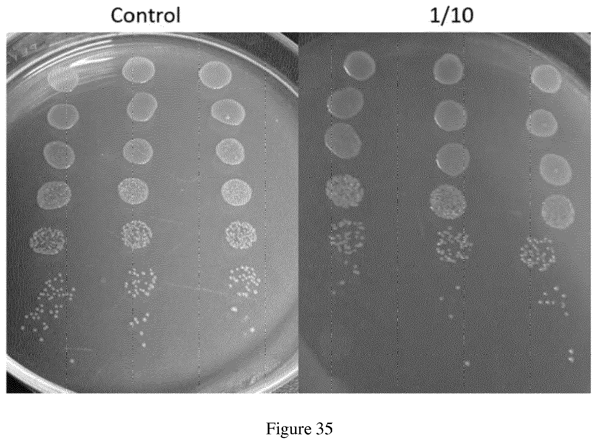

The MCB was established at the 1/10 fraction by counting the dilutions (Figure 35). 4.4×10⁹ UFC/mL were found in the control and 1/10 7,86×10⁸ UFC/mL in the 1/10 fraction, which resulted in a bactericidal action of 86,21% for the 1/10 dilution of Composition 4.

### Conclusions

Compositions 1, 2, 3, and 4 generated an inhibitory effect on *Aggregatibacter actinomycetemcomitans.*

Table 4 shows the effectiveness of the dilutions of each of the compositions of the present invention in inhibiting the growth of *Aggregatibacter actinomycetemcomitans.*

**Table 4**

| | **% of inhibition** |
|---|---|
| Composition 1 (1/60 dilution) | 99,1 |
| Composition 2 (1/70 dilution) | 80,6 |
| Composition 3 (1/60 dilution) | 83,3 |
| Composition 4 (1/60 dilution) | 82,8 |

100% inhibition was achieved with the 1/50 dilution for Compositions 1, 3, and 4 and the 1/60 dilution for Composition 2.

Table 5 summarizes the effectiveness of the dilutions of each of the compositions of the present invention in the bacterial elimination of *Aggregatibacter actinomycetemcomitans.*

**Table 5**

| | **% of elimination** |
|---|---|
| Composition 1 (1/10 dilution) | 91.1 |
| Composition 2 (1/10 dilution) | 85.3 |
| Composition 3 (1/10 dilution) | 91.6 |
| Composition 4 (1/10 dilution) | 86.2 |

### EXAMPLE 3

To evaluate whether there is a synergistic effect associated with the use of statins in combination with the rest of the active compounds of the compositions according to the present invention, assays were carried out with the compositions detailed in Table 6, which do not incorporate any type of statin.

**Table 6**

| **Active compound** | **Compos. 7 (% by weight)** | **Compos. 8 (% by weight)** | **Compos. 9 (% by weight)** | **Compos. 10 (% by weight)** | **Compos. 11 (% by weight)** |
|---|---|---|---|---|---|
| Peróxido de hidrógeno | 0,10 | - | - | 0,10 | - |
| Eugenol | 0.010 | 0.010 | - | - | 0.010 |
| Parachlorophenol | 0.004 | 0.004 | 0.004 | 0.004 | 0.004 |
| Methylparabeno | | | | | 0.05 |
| Camphor | 0.006 | 0.006 | 0.006 | 0.006 | 0.006 |
| Xylitol | 3.00 | 3.00 | 3.00 | 3.00 | 3.00 |

| | | | | | |
|---|---|---|---|---|---|
| Excipient used: Water in sufficient quantity. | | | | | |

### Assay 3.1: Evaluation of the antimicrobial activity against Fusobacterium nucleatum

To determine the antimicrobial activity of the compositions in Table 6 against *Fusobacterium nucleatum*, the methods described in Example 2 were followed.

### Results

A preliminary study on Compositions 7, 8, 9, 10, and 11 allowed for establishing which of them present some inhibitory activity. The result of this study was able to establish that only Compositions 5 and 8 present inhibitory activity against *Fusobacterium nucleatum.*

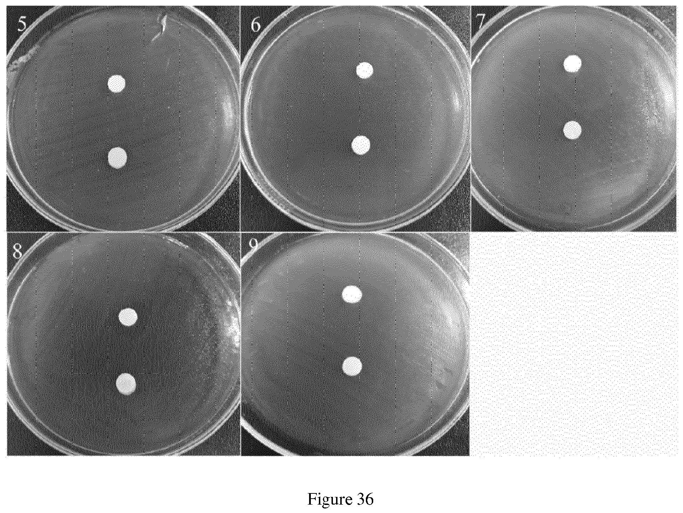

Figure 36 shows the antimicrobial testing of the compounds on *Fusobacterium nucleatum.* Plates with a strain of the bacteria, a sensidisc previously loaded with 10 µL and 20 µL of each composition placed over the culture can be observed.

### Determination of Minimum Inhibitory Concentration (MIC)

### Composition 7

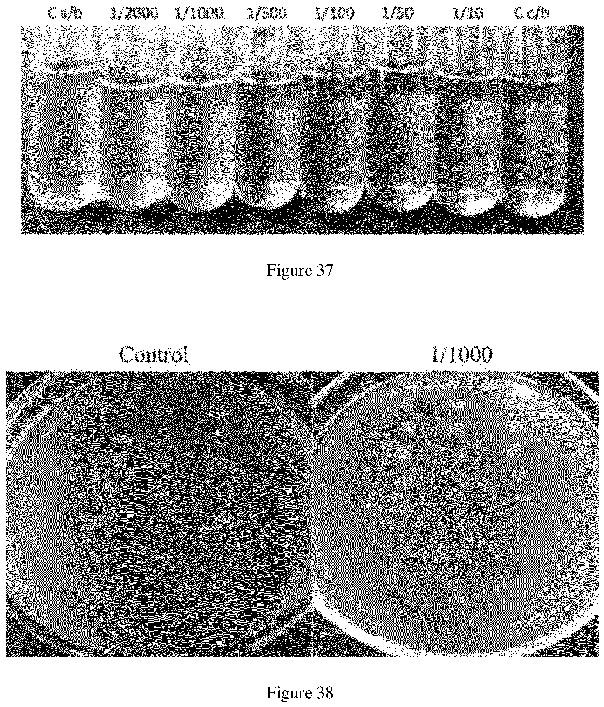

The serial dilutions of Composition 7 allowed the MIC to be established at 1/1000 (Figure 37), showing a variation in growth between 1/1000 and 1/500, with the latter having no growth.

The count of the control and 1/1000 solutions (Figure 38) showed that there is a clear decrease in growth, finding 8.4×10⁹ UFC/mL and 4.1×10⁸ UFC/mL, respectively, which results in an inhibition of 95.1% for the 1/1000 dilution of Composition 7.

### Composition 10

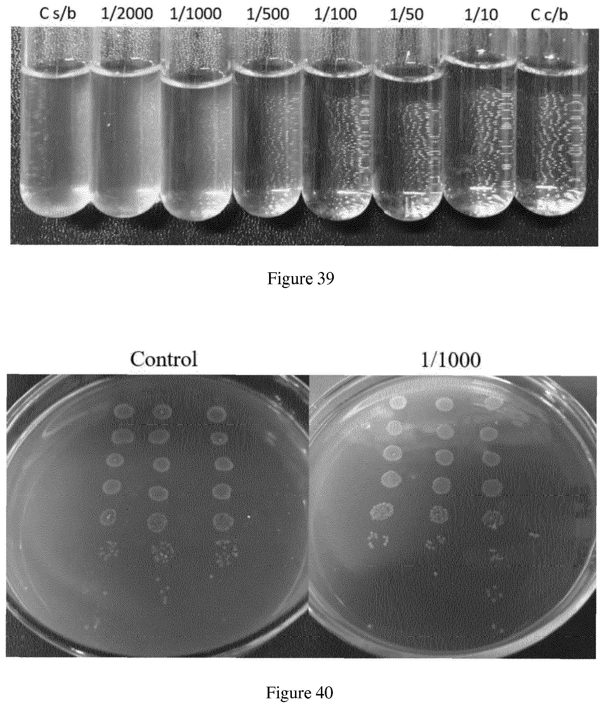

MIC was established at 1/1000 dilution (Figure 39), showing a variation in growth between 1/1000 and 1/500, the latter having non-existent growth.

Counting of control and 1/1000 solutions (Figure 40) showed that there is a clear decrease in growth, finding 8.4×10⁹ UFC/mL and 3.6×10⁹ UFC/mL, respectively, which allows establishing an inhibition of 42.8% for the 1/1000 dilution of Composition 10.

### Determination of the Minimum Bactericidal Concentration (MBC)

### Composition 7

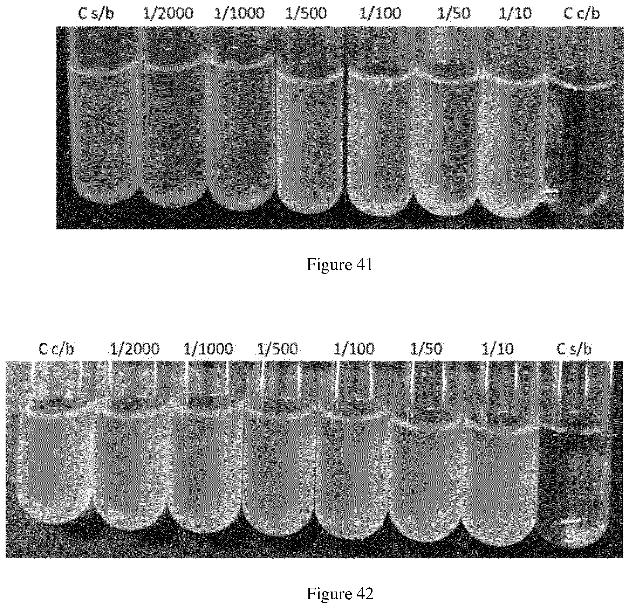

Serial dilutions of this composition from 1/10 to 1/2000 allowed the minimum bactericidal concentration to be established (Figure 41). The turbidity of the solutions indicated that bacteria were not affected regardless of the dilution.

### Composition 10

Serial dilutions of this composition from 1/10 to 1/2000 allowed for the establishment of the minimum bactericidal concentration (Figure 42). The turbidity of the solutions indicated that bacteria were not affected regardless of the dilution.

### Conclusions

Compositions 7 and 10 do not present a relevant inhibitory effect when the bacteria are in culture; Therefore, their development is not interrupted by said composition. Thus, it can be concluded that these compositions present a momentary effect that is not sustained over time.

### Assay 3.2: Evaluation of antimicrobial activity on Aggregatibacter actinomycetemcomitans

The antimicrobial activity against *Aggregatibacter actinomycetemcomitans* was determined for each of the compositions in Table 6, following the same methodologies described in Example 2. The preliminary study for all compositions was based on establishing whether the compounds have inhibitory activity. Through this experimentation, it was determined that only Compositions 7 and 9 present inhibitory activity on *Aggregatibacter actinomycetemcomitans.*

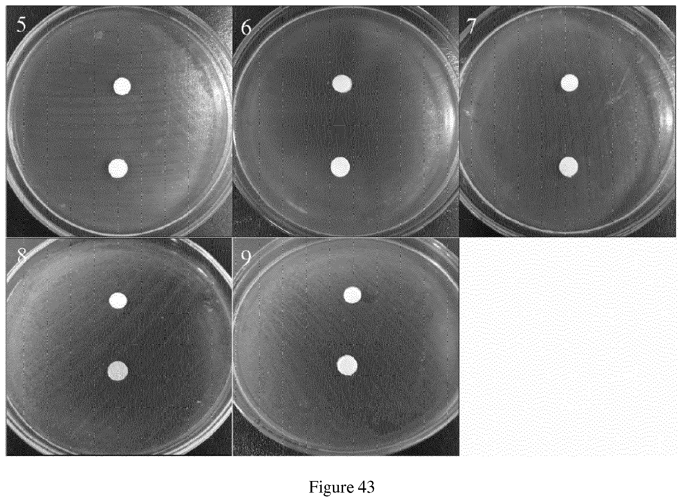

Figure 43 shows a lawn of the bacteria and a sense disc previously loaded with 10 µL and 20 µL of each composition placed on the culture.

### Determination of Minimum Inhibitory Concentration (MIC)

### Composition 7

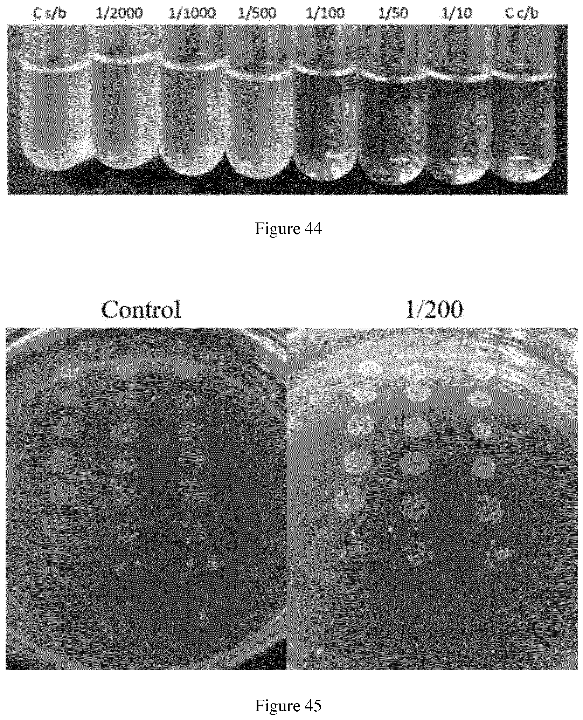

The preliminary study established the MIC within 1/500 and 1/100 dilutions (Figure 44), and the subsequent study of dilutions within said range showed growth up to the 1/200 fraction, having the 1/100 fraction of Composition 7 non-existent growth.

Counting control and 1/200 solutions (Figure 45) showed that there is a clear decrease in growth, finding 1.2×10¹¹ UFC/mL and 6.1×10⁹ UFC/mL, respectively, which resulted in an inhibition of 94% for the 1/200 dilution of Composition 7.

### Composition 9

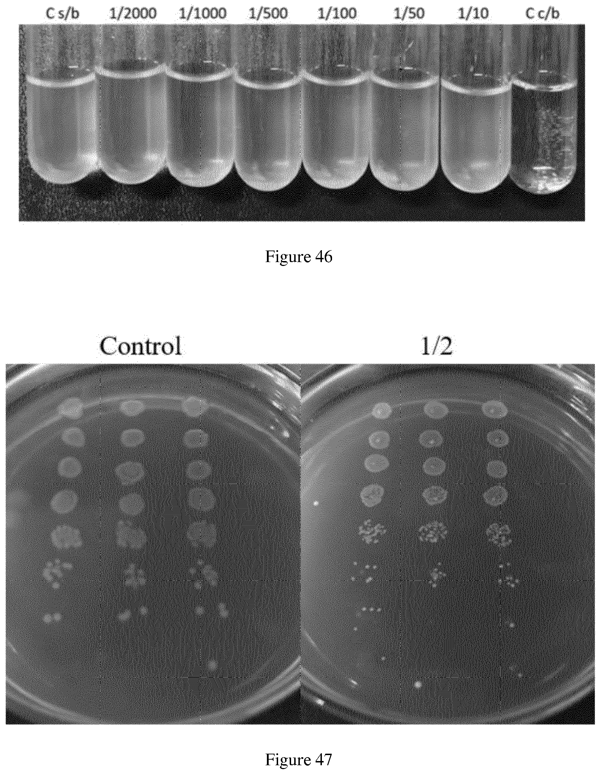

The dilution study of Composition 9 (Figure 46) showed growth up to the 1/2 fraction. Counting the control and 1/2 solutions (Figure 47) showed that there is a clear decrease in growth, finding 1.2×10¹¹ UFC/mL and 5.8×10⁸ UFC/mL, respectively, which allows establishing an inhibition of 99.51% for the 1/2 dilution of Composition 9.

### Determination of Minimum bactericidal concentration (MBC)

### Composition 7

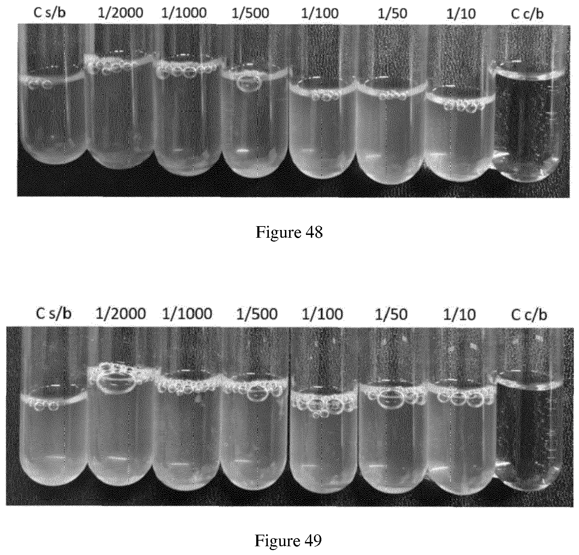

Serial dilutions of composition 7 from 1/10 to 1/2000 allowed the minimum bactericidal concentration to be established (Figure 48). The turbidity of the solutions indicated that bacteria were not affected regardless of the dilution.

### Composition 9

Dilutions of this composition from 1/10 to 1/2000 allowed for the establishment of the minimum bactericidal concentration (Figure 49). The turbidity of the solutions indicated that bacteria were not affected regardless of the dilution.

Compositions 7 and 9 do not present a relevant inhibitory effect when the bacteria are in culture; therefore, the composition does not interrupt its development. Thus, it can be concluded that these compositions present a momentary effect that is not sustained over time.

### EXAMPLE 4

In order to determine if statins alone at concentrations as low as 0.02% by weight have any bactericidal activity against bacteria that cause periodontitis (chronic and severe), tests were carried out with the following compositions:

**Table 7**

| **Composition** | **Active Ingredient (% by weight)** |
|---|---|
| A | Atorvastatin (0.02) |
| S | Simvastatin (0.02) |
| R | Rosuvastatin (0.02) |
| P | Pravastatin (0.02) |

### Assay 4.1: Antimicrobial activity against Fusobacterium nucleatum

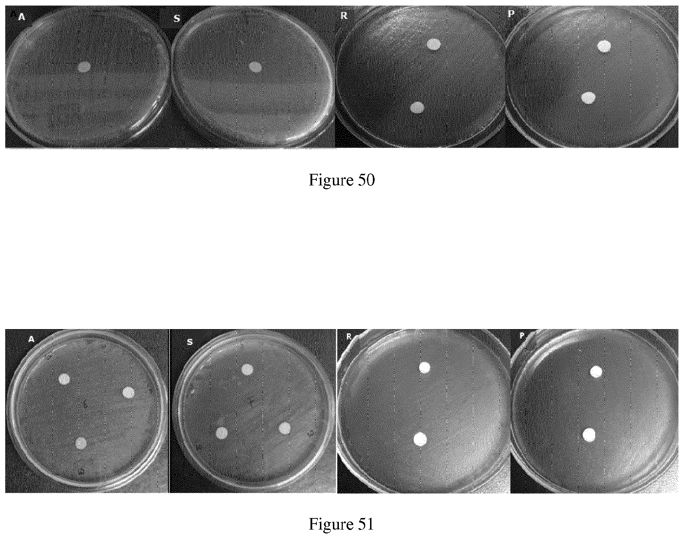

The preliminary study was based on establishing whether Compositions A, S, R, and P present inhibitory activity. Through this experimentation it was determined that none of these Compositions have inhibitory activity against *Fusobacterium nucleatum* (Figure 50).

In Figure P it is possible to see plates with a strain of the bacteria, a sensidisc previously loaded with 10 µL and 20 µL of each composition placed over the culture.

### Assay 4.2: Antimicrobial activity against Aggregatibacter actinomycetemcomitans

The preliminary study using Compositions A, S, R, and P allowed for establishing whether they have inhibitory activity at the concentrations indicated in Table 7.

It was determined that none of the Compositions A, S, R, and P present inhibitory activity against *Aggregatibacter actinomycetemcomitans* (Figure 51).

It is possible to see plates with a strain of the bacteria, a sensidisc previously loaded with 10 µL and 20 µL of each composition placed over the culture.

### EXAMPLE 5

In order to determine if statins together with auxiliary agents, have any bactericidal activity against bacteria that cause periodontitis (chronic and severe).

Table 8 shows the composition used.

**Table 8**

| **Composition 8** | **% by weight** |
|---|---|
| Pravastatin | 0.020 |
| Eugenol | 0.010 |
| Methylparaben | 0.010 |
| Xylitol | 3.0 |

### Assay 5.1: Antimicrobial activity against Fusobacterium nucleatum

The preliminary study was based on establishing whether the composition in Table 8 presents inhibitory activity. Through this experiment, it was determined that there was no inhibitory activity against *Fusobacterium nucleatum.*

### Assay 5.2: Antimicrobial activity against Aggregatibacter actinomycetemcomitans

The preliminary study using the compositions of Table 8 allowed the establishment of if these have inhibitory activity.

The results showed that at the concentrations used, there was no inhibitory activity against *Aggregatibacter actinomycetemcomitans* (Figure 51).

### CONCLUSIONS

The tests carried out show that Compositions 1, 2, 3, and 4, according to the present invention, have inhibitory and bactericidal activity against *Fusobacterium nucleatum* and *Aggregatibacter actinomycetemcomitans.*

Regarding the comparative compositions, Compositions 7, 8, 9, and 10, which do not contain statins, did not show a bactericidal effect against *Fusobacterium nucleatum* and neither against *Aggregatibacter actinomycetemcomitans.*

The evaluation of the antimicrobial activity of Compositions A, S, R, and P, which only include a statin at a concentration of 0.02% by weight, did not show an inhibitory effect on the bacteria mentioned above. While incorporating auxiliary agents into Composition P, but not an oxidizing antibacterial such as hydrogen peroxide, showed no activity against the bacteria studied either. From the studies carried out, it is evident that the compositions according to the present invention present a synergistic effect of inhibitory and bactericidal action against bacteria involved in chronic and severe periodontitis. The synergistic effect is attributable to the combination of statin-oxidizing antibacterial agents together with auxiliary agents that facilitate the action of the active compounds.

In fact, the compositions that lack the presence of statins (7, 8, 9 and 10), although some results of the studies showed inhibitory activity, they do not present an elimination effect on the bacteria that cause periodontitis. On the other hand, formulations that only include statins in a concentration of 0.02% by weight (A, S, R, and P), did not show any effect, neither inhibitory nor bactericidal, on the bacteria causing periodontitis.

Without being limited by theory, the inventors believe that the synergistic effect of the compositions could be attributed to the different mechanisms of action of each component of the composition that would act synergistically. Namely, the oxidizing and destructive effect of hydrogen peroxide on anaerobic bacteria would allow for increasing and improving the capacity of statins to inhibit the growth of bacterial biofilm through the interaction of methyl groups, or cyclopropyl rings with amino acid residues, reducing the formation of biofilms, in the presence of the facilitating agent(s) that contribute to the synergy of the active ingredients, making bacteria as resistant as *Fusobacterium nucleatum* and *Aggregatibacter actinomycetemcomitans,* unable to resist the antibiotic attack of the composition of the invention. This effect is demonstrated when the composition of the invention is effective, even in low concentrations of active ingredients, only in the presence of the facilitating agent or agents.

The results obtained become important when it is observed that it is possible to have an effect on the control and elimination of bacteria as aggressive as *Aggregatibacter actinomycetemcomitans,* at very low concentrations of each component, which provides great versatility to the formats in which these compositions can find.

The preceding specification is provided for illustrative purposes only and is not intended to describe all possible aspects of the present invention. While the invention has been shown herein and described in detail with respect to various exemplary embodiments, those skilled in the art will appreciate that minor changes to the description and various other modifications, omissions, and additions are made without departing from the spirit and scope thereof.

## Claims

1. Oral pharmaceutical composition, **CHARACTERIZED in that** it comprises at least one statin and at least one antiseptic and/or antibacterial agent.

2. The composition according to claim 1, **CHARACTERIZED in that** it further comprises at least one pharmaceutically acceptable auxiliary facilitating agent.

3. The composition according to claim 1, **CHARACTERIZED in that** the at least one antiseptic and/or antibacterial agent is an oxidizing compound.

4. The composition according to claim 1, **CHARACTERIZED in that** the at least one statin is fat soluble.

5. The composition according to claim 1, **CHARACTERIZED in that** the at least one statin is water soluble.

6. The composition according to claim 4, **CHARACTERIZED in that** the statin is selected from lovastatin, simvastatin, atorvastatin, and pitavastatin.

7. The composition according to claim 5, **CHARACTERIZED in that** the statin is selected from pravastatin, fluvastatin, and rosuvastatin.

8. The composition according to claim 1, **CHARACTERIZED in that** the at least one statin is present in an amount of at least 0.00029% by weight relative to the total of the composition.

9. The composition according to claim 3, **CHARACTERIZED in that** the at least one oxidizing antiseptic and/or antibacterial agent is selected from hydrogen peroxide and sodium hypochlorite, preferably hydrogen peroxide.

10. The composition according to claim 9, **CHARACTERIZED in that** the hydrogen peroxide is present in an amount of at least 0.0014% by weight relative to the total of the composition.

11. The composition according to claim 9 **CHARACTERIZED in that** the sodium hypochlorite is present in an amount of at least 0.0023% by weight relative to the total of the composition.

12. The composition according to claim 1, **CHARACTERIZED in that** the at least one auxiliary facilitating agent is a preservative derived from phenol.

13. The composition according to claim 12, **CHARACTERIZED in that** the preservative derived from phenol is selected from phenol, parachlorophenol, alkylparaben, and mixtures thereof.

14. The composition according to claim 13, **CHARACTERIZED in that** the parachlorophenol is present in an amount of at least 0.00006% by weight relative to the total of the composition.

15. The composition according to claims 13 y 14, **CHARACTERIZED in that** the parachlorophenol includes a carrier corresponding to camphor in an amount of at least 0.00009% by weight relative to the total of the composition.

16. The composition according to claim 13, **CHARACTERIZED in that** the alkylparaben is selected from methylparaben, ethylparaben, propylparaben, butylparaben, isopropylparaben, and isobutylparaben.

17. The composition according to claim 16, **CHARACTERIZED in that** the methylparaben is present in an amount of at least 0.00071% by weight relative to the total of the composition.

18. The composition according to claim 1, **CHARACTERIZED in that** the at least one auxiliary facilitating agent is an essential oil.

19. The composition according to claim 18, **CHARACTERIZED in that** the essential oil is selected from eugenol, camphor, and a mixture thereof.

20. The composition according to claim 19, **CHARACTERIZED in that** the eugenol is present in an amount of at least 0.00014% by weight relative to the total of the composition.

21. The composition according to claim 1, **CHARACTERIZED in that** the at least one auxiliary facilitating agent is a wetting agent.

22. The composition according to claim 19, **CHARACTERIZED in that** the wetting agent is xylitol.

23. The composition according to claim 22, **CHARACTERIZED in that** the xylitol is present in an amount of at least 0.04% by weight relative to the total of the composition.

24. The composition according to claim 1, **CHARACTERIZED in that** the at least one auxiliary facilitating agent is a diluent.

25. The composition according to claim 24, **CHARACTERIZED in that** the diluent is purified water.

26. The composition according to claim 1, **CHARACTERIZED in that** further comprises a pH regulator (buffer).

27. The composition according to claim 26, **CHARACTERIZED in that** the pH regulator is a bicarbonate buffer.

28. The composition according to claim 1, **CHARACTERIZED in that** it further comprises flavoring agents, sweeteners, or a mixture thereof.

29. Pharmaceutical formulation, **CHARACTERIZED in that** it comprises a composition according to claim 1, wherein said formulation is selected from toothpaste, gel, mouthwash, dental floss, tablets to dissolve in the mouth and controlled release intraoral elements or devices such as microparticle or nanoparticle capsules.

30. Use of the composition according to claim 1, **CHARACTERIZED in that** it is useful in the manufacture of a medicament to prevent and/or treat diseases of the soft and hard tissues surrounding the tooth present in the oral cavity.

31. The use according to claim 29, **CHARACTERIZED in that** the disease of the soft and hard tissues surrounding the tooth is selected from gingivitis and periodontitis.

32. The use according to claim 31, **CHARACTERIZED in that** the periodontitis is selected from chronic periodontitis and severe periodontitis.

33. Pharmaceutical kit for oral use, **CHARACTERIZED in that** it comprises the composition according to claim 1 and instructions for use.

34. Method to prevent or treat diseases of the soft and hard tissues surrounding the tooth present in the oral cavity, **CHARACTERIZED in that** it comprises administrating an oral pharmaceutical composition according to claim 1.

35. The method according to claim 34 **CHARACTERIZED in that** the disease of the soft and hard tissues surrounding the tooth is selected from gingivitis and periodontitis.

36. The method according to claim 35, **CHARACTERIZED in that** the periodontitis is selected from chronic periodontitis and severe periodontitis.

37. Oral pharmaceutical composition according to claim 1, **CHARACTERIZED in that** it is used to prevent and/or treat diseases of the soft and hard tissues surrounding the tooth present in the oral cavity.

38. The oral pharmaceutical composition according to claim 36, **CHARACTERIZED in that** the disease of the soft and hard tissues surrounding the tooth is selected from gingivitis and periodontitis.

39. The oral pharmaceutical composition according to claim 38, **CHARACTERIZED in that** the periodontitis is selected from chronic periodontitis and severe periodontitis.
